# EUROPEAN PATENT APPLICATION

(11) **EP 4 725 480 A1**
(43) Date of publication of application: **15.04.2026**
(21) Application number: 24315472.1
(22) Date of filing: 14.10.2024
(51) Int. Cl.: A61K 31/197, A61K 31/198, A61K 31/409, A61K 45/06, A61P 35/00, G01N 33/00

(54) **RAMAN SPECTROSCOPY FOR PERSONALIZED CANCER MEDICINE**

(71) Applicant: Endress+Hauser Process Analysis Support SARL, 69800 Saint-Priest (FR); CENTRE LEON BERARD, Regional Comprehensive Cancer Center, 69373 Lyon Cedex 08 (FR); Université Claude Bernard Lyon 1, 69622 Villeurbanne (FR); INSERM TRANSFERT, Société Anonyme à Directoire et Conseil de Surveillance, 75013 Paris (FR); CENTRE NATIONAL DE LA RECHERCHE SCIENTIFIQUE, 75794 Paris Cedex 16 (FR)
(72) Inventor: Lambert Angulo, Leticia, 69970 Chaponnay (FR); Cosset, Erika, 38110 Sain-Didier de la tour (FR); Lambert Angulo, Leticia, 69970 Chaponnay (FR)
(74) Representative: Endress + Hauser Group Services (Deutschland) AG+Co. KG

(57) **Abstract**

A kit for analyzing anti-tumor effect of a pharmaceutical composition inhibitor comprising
(i) a Raman spectrometer comprising a Raman probe
(ii) optionally a cell imaging apparatus,
(iii) a composition comprising an anti-tumor agent,
(iv) a single or a multi-well plate, wherein the multi-well-plate preferably is selected from a 24-, 48-, or a 96-well plate.

## Description

One in five people worldwide will be diagnosed with cancer in their lifetime. Nowadays, chemotherapy is the standard cancer treatment (International Agency for Research on Cancer 2023). In many cases, standard cancer treatment is ineffective when cancer cells develop resistance, or a side effect occurs from drug administration. Despite advancements in neuroimaging, neurosurgery, chemotherapy (i.e., temozolomide for brain cancer, TMZ), and radiotherapy, the heterogeneity of brain tumors (glioblastoma) contributes to poor patient outcomes, with an overall survival of about 15 months.

On the contrary, drugs that target specific characteristics of tumors (type of cells that are present, genes and proteins) can be highly effective without affecting normal cells; therefore, they can increase life expectancy and reduce side effects.

Cancer stem cells play a crucial role in tumor formation. Not only can they self-renew, but they also give rise to other cell types within the tumor. Genomic studies have provided extensive data on glioblastoma (GBM) and its microenvironment, revealing several molecular drivers, but these findings have not yet been effectively translated into clinical practice. Challenges include tumor heterogeneity, infiltrative nature, cancer stem cells, redundant signaling pathways, necrotic features, and drug delivery across the blood-brain barrier. Particularly important, Glioblastoma Stem Cells (GSCs) are crucial for tumor propagation, drug resistance, and relapse.

Recent research focuses on specifically eliminating cancer stem cells rather than targeting all tumor cells. Leukemia, breast cancer, skin cancer, brain cancer, among other cancer types, can be treated using this new approach.

On the other hand, mutations in the TP53 gene, the gene that encodes the p53 protein, is the most frequently mutated gene in tumors accounting for 50% of human cancers (Prives and Hall, 1999, J. Pathol. 186:112-26; Hollstein et al., 1991, Science 253:49-53; Vogelstein et al., 2000). The p53 protein is also called tumor protein P53, cellular tumor antigen p53, or transformation-related protein 53 (TRP53) and its mutation causes checkpoint defects, uncontrolled cell proliferation, genomic instability, and tumorigenic mutations.

To harness cancerous tumor vulnerabilities, this invention targets: i) the self-renewing, differentiating and resistant nature of cancer stem cells; and ii) the common p53 mutation, by proposing the usage of specific small molecules. To determine the efficient dosage of these treatments, Raman spectroscopy will be used for tumor staging (i.e., to evaluate the cancer progression or stage of cancer), and the treatment dose will be calculated accordingly.

Protoporphyrin IX (PpIX) is considered a small molecule which can cross the blood-brain barrier, and it does not cause toxicity in normal cells. During cellular respiration, 5-aminolevulinic acid (5-ALA) is naturally converted into PpIX in the mitochondria. PpIX is then transformed into heme when a ferrous iron is added to the PpIX molecule. Heme is a critical component of hemoglobin which delivers oxygen to cells for energy production and removes waste products like CO2, maintaining metabolic balance. However, higher concentrations of PpIX and heme are found in tumors due to the high metabolic activity in the cell division process (mitosis).

In consequence, monitoring the concentration of PpIX, heme and their ratio (PpIX / Heme) within stem cells also provides insights into tumor development time (i.e., progression of cancer), as elevated levels of both compounds, PpIX and Heme, correlate with higher tumor grade.

The TP53 gene, in response to several stressors, including DNA damage, hypoxia and oncogenic activation, activates p53 to promote cell cycle arrest, apoptosis or senescence thereby suppressing tumor growth. It also plays many additional roles including regulating cellular metabolism (Muller et al., 2009). Moreover, TP53 activation also initiates a number of DNA repair pathways (Fei and El'Deiry, 2003, Oncogene 22:5774-83).

In cancer cells with a mutated p53, the tumor suppressor protein p73 is inactivated. Mouse double minute 2 homolog MDM2 or E3 ubiquitin-protein ligase is a negative regulator of the p53 and p73 tumor suppressors that binds to the inactive p73 and the mutant p53, whereby the three molecules form a complex. A further molecule, which attaches to p73 is the E3 ubiquitin-protein ligase ITCH. It ubiquitinylates p73 and thereby catalyzes its degradation.

In this respect, PpIX has been demonstrated to significantly inhibit specific protein interactions involved in cancer. For example, PpIX disintegrates the complex including MDM2 and mutated or intact p53, which leads to an improved regulation of cell proliferation. In cancer cells with mutated p53, PpIX also interacts with p73, releasing p73 from MDM2, and hereby allowing p73 to reactivate its antitumor properties. Hence, PpIX triggers accumulation of p73, and inhibits its disintegration.

PplX also fulfills a pro-oxidant function, by inhibiting thioredoxin reductase (TrxR) and elevating Reactive Oxygen Species (ROS) level for the induction of apoptosis (cell death) in cancer.

Beyond that, PplX targets the self-renewing and differentiating nature of glioblastoma stem cells.

Compositions, which are suitable for the treatment of brain tumors, are restricted, as the blood-brain-barrier prevents large protein-based pharmaceuticals, and biologicals, from entering the brain. Therefore, there is a need for compositions, which pass through the blood-brain barrier for the treatment of brain tumors.

In particular, there is an urgent need for compositions, which effectively destroy glioblastoma tumors, particularly the resistant, self-renewing and differentiating nature of glioblastoma stem cells, and cancer cells that have a p53 mutation. It is desired that these compositions have less or no adverse side effects of commonly used chemotherapeutics. Also, it is desired to provide compositions, which can pass the blood-brain-barrier to target brain tumors.

Elevating Reactive Oxygen Species (ROS) levels can be also achieved by the inhibition of Taurine Transporter (TauT) - a membrane protein that transports taurine into the cell. Taurine is a natural antioxidant in the body. Lack of taurine has been shown to impair the transfer of electrons in mitochondria and to increase the production of free radicals. Besides, taurine also suppresses enzymes responsible for ROS generation. With no taurine stimulating antioxidant activity, there will be an increase in the ROS levels up to a point that mutant p53 cannot tolerate, since it is vulnerable to oxidation; thus, the cell being forced to undergo apoptosis. This oxidation can also be effective for other cell lines that, despite lacking the mutation in p53, are highly sensitive to oxidative environments.

TauT inhibitors exist, which - like PpIX - are also small molecules capable of passing the blood-brain barrier. These components include taurine analogues such as piperidine-4-sulfonic acid, g-Aminobutyric acid, g-vinyl-aminobutyric acid, β-alanine, and GABA-mimetics such as gaboxadol or 4,5,6,7-tetrahydroisoxazolo(5,4-c)pyridin-3-ol (THIP), nipecotic acid, and guvacine or any pharmaceutically acceptable salt thereof. Further TauT inhibitor include polypeptide-based antibodies, as shown in US 20190202885 A1. The advantage of TauT inhibitors is that they are water-soluble, as well as being soluble in organic solvents.

The object of this invention is to provide anti-tumor compositions for use in cancer treatment that are less toxic than commonly used chemotherapeutic agents and that pass the blood-brain-barrier. A further aspect of the invention is the provision of an analysis method, which allows for determining the effective concentration and ratio of the components required to reduce tumor growth, which may be applied in real-time, and which is conducted on living cells. Furthermore, the object of the invention is the provision of a method, which allows for an analysis of the efficacy of a tumor-inhibiting agent in real time, covering several parameters at the same time.

The first aspect of the invention relates to a composition comprising Protoporphyrin IX (PpIX) or a pharmaceutically acceptable salt thereof and a pharmaceutical excipient, wherein preferably the excipient is a carrier or a diluent.

Beyond its use for fluorescence-guided brain resection, PpIX derived from 5-ALA has therapeutic uses, when activated by light, ultrasound, or radiation, it produces ROS within the mitochondria leading to death of some of the tumor cells while sparing most healthy tissue. However, achieving sufficient ROS from 5-ALA administration to -fully eradicate cancer cells remains a challenge, limiting its effectiveness as a therapeutic method.

Conversely, using Raman Spectroscopy to predict the amount of PpIX / Heme that that is naturally present as part of cellular metabolism of stem cells to carry out mitosis and precisely administering an additional dose of PpIX can effectively eliminate cancer stem cells without harming healthy tissue - as healthy cells don't undergo mitosis. In summary, PpIX and heme play a crucial role in stem cell metabolism, but extra levels of PpIX can indicate the cancer cells that its natural conversion into heme is not happening correctly, thus, forcing the cells to undergo apoptosis.

In a second aspect, the invention provides a further composition according to the first aspect for use in treating of cancer, wherein the composition for the use is to be administered to a cancer patient to inhibit the growth of cancer cells, in particular cancer stem cells.

In one embodiment, the cancer is a brain tumor, a glioblastoma, a pancreatic tumor, a kidney tumor, colon carcinoma, osteosarcoma, or wherein the cancer is leukemia.

The tumor is preferably a human tumor.

In one embodiment of the composition according to the first aspect of the invention or the composition for the use according to the second aspect of the invention or an embodiment thereof the concentration of PpIX is selected from 1-10 µg/ml, preferably 10 µg/ml.

Another, third aspect of the invention relates to a composition comprising a TauT inhibitor or a pharmaceutically acceptable salt thereof for use in treating cancer, wherein the composition is to be administered to a cancer patient to inhibit the growth of cancer cells, in particular cancer tumor cells having a mutation in the TP53 gene or cancer tumor cells lacking a mutation in the TP53 gene, wherein the cancer tumor cells lacking a mutation in the TP53 gene are more vulnerable to ROS-induced damage or/and damage induced by Reactive Nitrogen Species (RNS). This includes cancer cells exhibiting high levels of oxidative and nitrosative stresses due to their rapid growth and metabolic activity, for example, glioblastoma cell lines, such as the Ge518.

In one embodiment of the composition comprising a TauT inhibitor for the use in treating cancer, wherein the TauT inhibitor is selected from piperidine-4-sulfonic acid, g-Aminobutyric acid, g-vinyl-aminobutyric acid, β-alanine, 2-Amino-2-methyl-1-propanol (AMP), or any pharmaceutically acceptable salt thereof, preferably piperidine-4-sulfonic acid or a pharmaceutically acceptable salt thereof, and 5-aminolevulinic acid (5-ALA) or a pharmaceutically acceptable salt thereof.

2-Amino-2-methyl-1-propanol (AMP)I, for example, is dissolved in dimethylsulfoxide (DMSO) or in methylsulfonylmethane (MSM).

In one embodiment of the composition comprising a TauT inhibitor for the use in treating cancer, the concentration of the TauT inhibitor, preferably piperidine-4-sulfonic acid, is selected from 0.15 mM to 15 mM.

In one embodiment of the composition comprising a TauT inhibitor for the use in treating cancer, the concentration is selected from 0.15, 0.3, 1.25, 2.5, 5, 10, 12.5 and 15 mM.

In one embodiment the composition of the first aspect of the invention, the composition for the use according to the second aspect or an embodiment thereof or the third aspect or an embodiment thereof comprises a pharmaceutical excipient, wherein preferably the excipient is a carrier or a diluent. Preferably, the carrier or diluent is configured to pass the blood-brain barrier.

Many pharmaceutical excipients are described in detail in the Handbook of Pharmaceutical Excipients, published jointly by the American Pharmaceutical Association and The Pharmaceutical Society of Great Britain (The Pharmaceutical Press, 1986).

Suitable carriers are e.g. disclosed in US 2015212086 A1. Suitable carriers are vectors for targeting molecules across the blood brain barrier are known in the art. For example, lentiviral vectors (including a gene of interest) that can cross the blood brain barrier and method of making them are disclosed for example in US. Pat. No. 7,090,837. Other possible carriers include nanoparticles, liposomes, micelles and conjugates of the active agent, such as PpIX and p4S with a solubility enhancing molecule.

Suitable diluents are well known to those skilled in the art, and include materials such as carbohydrates, waxes, water soluble and / or swellable polymers, hydrophobic materials, gelatin, oils, solvents, water and the like. The particular carrier or diluent used will depend on the the hydrophilic or hydrophic properties of the molecule, the manner and purpose for which the compound of the invention is applied. Solvents are generally selected based on solvents recognized by those skilled in the art to be safe for administration to a mammal, i.e. generally recognized as safe (GRAS) by the Food and drug administration (FDA). Suitable aqueous solvents include water, ethanol, propylene glycol, polyethylene glycols (eg PEG 400, PEG 300) and the like and mixtures thereof.

Suitable non-aqueous solvents comprise DMSO (dimethylsulfoxide) or methylsulfonylmethane (MSM). DMSO and MSM are especially suitable for active agents, which have a poor solubility in water, such as PpIX. Furthermore, MSM passes the blood-brain barrier, which is required for a formulation used in the treatment of brain tumors. In addition, MSM is generally recognized as safe by the food and drug administration.

The compositions described herein are configured to be administered using several routes of administration. The compounds and pharmaceutical compositions described herein can be administered to the subject in a number of ways depending on whether local or systemic treatment is desired, and on the area to be treated. Examples of routes of administration include parenteral, e.g., intravenous, intradermal, subcutaneous, oral (e.g., inhalation) and transmucosal. Parenteral administration, if used, is generally characterized by injection. Injectables can be prepared in conventional forms, either as liquid solutions or suspensions, solid forms suitable for solution or suspension in liquid prior to injection, or as emulsions.

In one embodiment, the orally to be administered composition is comprised in a liquid, the composition is comprised in tablets, in capsules.

In one embodiment, the dose of the composition for the use according to the second aspect or an embodiment thereof or the third aspect or an embodiment thereof is to be administered 7 weeks after surgery in which most of the tumor has been removed.

In one embodiment, the dose of the composition for the use according to the second aspect or an embodiment thereof or the third aspect or an embodiment thereof is wherein the composition is to be repeated for at least 6 weeks, preferably for at least 2 months, more preferably for at least 6 months.

In one embodiment, the administered composition is combined with radiotherapy or photodynamic therapy. Preferably, radiotherapy is carried out over a period of 6 weeks, wherein the radiotherapy is initiated in the beginning of the administration regimen.

In one embodiment, the cancer cells or tumor cells have a mutation in the TP53 gene, wherein the mutation results in an altered expression of a p53 translation product and/or in expression of a non-functional p53 protein that corresponds to an alteration in a human p53 gene associated with tumor growth or wherein the cancer cells retain intact TP53 gene

Preferably, the composition for the use according to the second aspect or any embodiment thereof or the third aspect or any embodiment thereof, the cancer cells are tumor cells that have a mutation in the TP53 gene, wherein the mutation results in an altered expression of a p53 translation product and/or in expression of a non-functional p53 protein that corresponds to an alteration in a human p53 gene associated with tumor growth.

In an alternative embodiment, the cancer cells retain an intact p53 gene. This pertains to cells that are, nonetheless, vulnerable to ROS-induced damage, for instance, glioblastoma cells exhibiting high levels of oxidative stress due to their rapid growth and metabolic activity.

Raman spectroscopy is a spectroscopic method, which is used to determine vibrational modes of molecules, although rotational and other low-frequency modes of systems may also be observed. Raman spectroscopy is commonly used in chemistry to provide a structural fingerprint by which molecules can be identified.

A Raman spectrum provides an instantaneous spectral coverage in a wide Raman shifts range to capture well-defined Raman peaks of the components present in the cell culture. These peaks are directly translated into important process information (i.e., concentrations, changes over time).

Raman spectroscopy allows real time measurements for quantifiable compositional, chemical, and physical properties of a sample on living cells in a non-destructive way within the cell culture in a bioreactor without the need of any sample preparation, which are otherwise acquired via offline via assay measurements. Spectral preprocessing, along with multivariate analysis, such as PLS, PCA, ML, DL, ANN and/or IHM, for example, provides information that may be plotted on a time scale to inform and alert an operator, in real time, of the quantifiable and/or the relative status of important constituents during the bioprocess.

A further aspect of the invention relates to a kit for analyzing anti-tumor effect of a pharmaceutical composition inhibitor comprising
(i) a Raman spectrometer comprising a Raman probe
(ii) optionally a cell imaging apparatus,
(iii) a composition comprising an anti-tumor agent,
(iv) a single or a multi-well plate, wherein the multi-well-plate preferably is selected from a 24-, 48-, or a 96-well plate.

In one embodiment of the kit, the Raman probe is configured to automatically screen a single- or a multi-well-plate, wherein the Raman probe is connected to a transport mechanism that is electrically connected to a controller configured to move the probe relative to the single- or multi-well-plate in a horizontal plane along an x-, y-axis and vertically along the z-axis.

In alternative embodiment of the kit, the multi-well plate is configured to move in a horizontal plane along an x- and y-axis and vertically along the z-axis, while the Raman probe is at a fixed position.

In one embodiment of the kit the tumor cells, preferably glioblastoma cells, are contacted with PpIX, wherein the excitation wavelength at which the PpIX is excited is at 785 nm or at 993 nm, preferably at 993 nm.

In one embodiment the tumor-reducing agent of the kit is selected from PpIX, preferably having a concentration of 1-10 µg/ml, preferably 10 µg/ml or a TauT inhibitor, wherein the TauT inhibitor is selected from from piperidine-4-sulfonic acid, g-Aminobutyric acid, g-vinyl-aminobutyric acid, β-alanine, 2-Amino-2-methyl-1-propanol (AMP), or any pharmaceutically acceptable salt thereof, preferably piperidine-4-sulfonic acid or a pharmaceutically acceptable salt thereof preferably having a concentration between 0.15 mM to 15 mM.

A further aspect of the invention relates to an *in vitro* method for monitoring the efficacy of anti-tumor growth therapy in real-time, wherein the method comprises:
providing a sample comprising tumor cells, preferably glioblastoma tumor cells, from a patient and cultivated as a cell culture, preferably having a mutation in the TP53 gene,
wherein the mutation results in an altered expression of a p53 translation product and/or in expression of a non-functional p53 protein that corresponds to an alteration in a human p53 gene associated with tumor growth;
contacting the tumor cells with a taurine transporter (TauT) inhibitor, wherein the taurine transporter inhibitor is selected from piperidine-4-sulfonic acid, g-Aminobutyric acid, g-vinyl-aminubutyric acid, β-alanine, 2-Amino-2-methyl-1-propanol (AMP), or any pharmaceutically acceptable salt thereof, preferably piperidine-4-sulfonic acid or a pharmaceutically acceptable salt thereof,
contacting the tumor cells with protoporphyrin IX (PpIX) or a pharmaceutically acceptable salt thereof, and/or
contacting the tumor cells with an anti-cancer drug selected from a chemotherapeutic agent, and
and acquiring continuous Raman spectra of the cancer cell culture samples.

In one embodiment of the kit, the Raman spectra are acquired at an interval, which lies between 1 minute and 2 hours, preferably between 1 minute and 20 minutes, more preferably between 1 minute and 5 minutes.

In some embodiments of the method a Raman analyzer with a 785 nm laser is used, the spectral coverage ranges between 150 - 3425 cm-1, and the resolution is 4 cm-1 or lower. In other embodiments of the method a Raman analyzer with a 993 nm laser is used (for comparison with the 785 nm one), wherein the spectral coverage ranges between 200-2400 cm-1, and the resolution is 5 cm-1 or lower. At 993 nm it is possible to reduce fluorescence in the Raman signal.

The advantage of analyzing by Raman as compared to the analysis by photometry lies in the fact that PplX is fluorescent, analyzing by photometry in the visible range (Vis) destroys the PpIX by bleaching. Analyzing at 993 nm does not destroy the sample, and therefore allows for the continuous analysis of the sample by Raman spectroscopy. In fact, Raman is advantageous over any analysis method, where a fluorescent sample, prone to bleaching is to be measured. Besides, using an excitation wavelength of 993 nm instead of 785 nm can significantly reduce fluorescence interference in the spectra, which often masks Raman signals. This reduction in fluorescence enhances the clarity and intensity of Raman peaks, facilitating more accurate and straightforward analysis of all components in the cell culture.

One aspect of the invention therefore relates to the *in vitro* method of analyzing the effect of a compound on the vitality of cell samples, wherein the compound or a cell molecule to be analyzed is autofluorescent wherein the analysis is carried out by Raman spectroscopy.

In one embedment, the in vitro method for the analysis of the efficacy of anti-tumor agent on growth of cancer cells when contacting the cancer cells with PpIX further comprises
applying analysis of the protein expression levels of one or more than one of
(a) Protoporphyrin IX (PpIX)
(b) active p73
(c) mutant p53
(d) reactive oxygen species, and
(e) heme.

In one embodiment, the method further comprises: calculating the ratio of Protoporphyrin IX (PpIX) and Heme (i.e., PpIX / Heme) in the cells.

In one embedment, the effect of the anti-cancer agent, particularly PpIX, on tumor cell growth, in particular, glioblastoma growth is determined after 5-7 days, and based on the effect, another dose of PpIX is applied, which is in the range of 1-10 µg/ml.

In one embedment, the in vitro method for the analysis of the efficacy of anti-tumor agent on growth of cancer cells when contacting the cancer cells with Taut inhibitor further comprises:
applying analysis of the protein expression levels of one or more than one of
(a) active p73
(b) mutant p53
(c) reactive oxygen species
(d) taurine transporter inhibitor
(e) taurine
(f) reactive nitrogen species (RNS)

In one embodiment, the method further comprises:
(v) determining the amount of complex 1 consisting of PpIX/ mutant p53/ p73 complex and/or
(vi) determining the amount of complex 2 consisting of MDM2/mutant p53/p73 complex
(vii) calculating the ratio of complex 1 and complex 2, wherein
increased ratio of complex 1 to complex 2 is indicative of an antitumor effect of the composition of any one of the invention or an embodiment thereof or the composition for the use of the invention or an embodiment thereof.

In one embodiment, the *in vitro* method further comprises acquisition of offline assay measurements of each of the complex 1, complex 2, and any one of compounds (a)-(f), concurrently to acquiring Raman spectra of the cancer cell culture cell sample.

Based on the offline assay measurements, which are used for referencing purposes of the composition and respective analyte concentrations of the measured samples, a correlative model of one or more than one target analytes is generated as a training data set, such that spectral changes in the training data set correlate with the offline measurements of the target analyte properties and/or compositions.

After having acquired the offline measurement of the target analytes and the sample set, a correlative model to process the data set to qualitatively and/or quantitatively predict a value of a property and/or composition of the target analyte.

In an additional embodiment, Raman spectra of each target analyte is acquired prior to an/or after the online Raman measurements.

Analysis of the formation of complexes in living cells is, e.g., carried out by fluorescent two-hybrid (F2H) Analysis in living cells. The rationale for the F2H assay is based on the fact that proteins are freely roaming the cell unless interactions with other cellular components immobilize them at specific structures. The method comprises tethering of fluorescently labeled MDM2 to the nucleus and subsequent analysis by confocal microscopy to assess complex formation as described by (Zolghadr et al., 2012, Methods Mol. Biol. 812:275-282).

In some embodiments, the Raman spectra are acquired by a Raman system including a Raman probe, which is configured and arranged to transmit excitation light into the bioprocess vessel under test to collect a Raman signal emitted from a sample volume within the bioreactor.

The Raman probe may be coupled to an excitation light source by a fiber optic cable such that excitation light emitted by the light source is incident upon a sample within the sample volume via a process connection. In at least one embodiment, the process connection includes an opening and is configured to enable attaching the probe to the reactor vessel such that the probe is in direct contact with the sample via the opening of the process connection. In a further embodiment, the process connection may include a window that is transparent to the excitation light of the light source such that the probe is in indirect contact with the sample via the window of the process connection.

In one embodiments of the method, the Raman spectrum is carried out by a Raman flow assembly, comprising: The Raman Rxn-10 probe that transmits the Raman signal to the analyzer along an optical fiber. The micro flow cell that contains the cell culture and gives a significant amplification of the collected Raman signal, wherein the flow cell has a total volume of around 2-20 ml, preferably 4-18, more preferably 12-14 ml.

Yet another aspect of the invention relates to a method of monitoring the efficacy of an anti-tumor agent, wherein the method comprises:
Providing a blood sample from a blood vessel of a tumor, preferably a glioblastoma tumor,
contacting the blood sample with
a taurine transporter (TauT) inhibitor, wherein the taurine transporter inhibitor is selected from piperidine-4-sulfonic acid, g-Aminobutyric acid, g-vinyl-aminubutyric acid, β-alanine, 2-Amino-2-methyl-1-propanol (AMP), or any pharmaceutically acceptable salt thereof, preferably piperidine-4-sulfonic acid or a pharmaceutically acceptable salt thereof,
protoporphyrin IX (PpIX) or a pharmaceutically acceptable salt thereof, and/or
an anti-cancer drug selected from a chemotherapeutic agent, and
and acquiring continuous Raman spectra of the blood sample.

In one embodiment, the blood is human blood.

Any one embodiment described herein is contemplated as being combinable with any one or more of the other embodiments, where applicable and not specifically disclaimed, even though the embodiments are described in different aspects of the present invention.

Raman spectroscopy has the advantage that once it has been established, there is no need for additional spectroscopic methods, and a comntinuous analysis of the sample is possible

In one embodiment, the method further comprises acquiring a mass spectrum of the cancer cell culture cells.

For analyzing the cell viability in a cell culture, state-of-the-art techniques are used.

One state-of the art technique is based on Trypan blue staining.

Trypan Blue, a diazo dye and a vital stain, has been used to selectively color dead tissues or cells blue. Live cells or tissues with intact cell membranes are not colored. Trypan Blue is not absorbed in a viable cell because cells are selective in the compounds that pass through the membrane. However, Trypan Blue traverses the membrane in a dead cell and stain dead cells with a distinctive blue color under a microscope.

Multi-sample cell count and viability analysis method commonly utilizes an image-based platform with automated liquid handling for mixing cell sample with Trypan Blue. The method uses one fixed focal plane to count live cells and Trypan Blue-stained dead cells and generates viability measurement.

In some embodiments, the method further comprises:
determining the viable cell count,
determining the total cell count and
calculating cell viability percentage based on viable cell count and total cell count.

Another state-of-the-art technique is flow cytometry. Flow cytometry is a technique used to detect and measure physical and chemical characteristics of a population of cells or particles.

In this process, a sample containing cells or particles is suspended in a fluid and injected into the flow cytometer instrument. The sample is focused to ideally flow one cell at a time through a laser beam, where the light scattered is characteristic to the cells and their components.

Cancer cells lines, which are used for the method include rat glioma cell lines (9L and C6), human glioma cell lines (U251 and T98G), human pancreatic cancer cell lines: Paca3 (wt TP53), MiaPaca2 (mutant TP53R248W), Panc1 (mutant TP53R273H), Panc02 (wt TP53), normal human pancreatic ductal epithelial cells (HPDE) immortalized by HPV transformation, human embryonic kidney 293 cells (HEK 293), Pig Kidney Epithelial Cells (LLC-PK1), MDCK (Madin-Darby Canine Kidney), Jurkat cells, Marine fibroblast, and MCF-7, EHEB (wt-p53) chronic B cell leukemia cells, HL-60 (p53-null) acute promyelocytic leukemia cell line, p53-defcient human colon HCT116 cancer cells, human non-small cell lung carcinoma cell line H1299, and osteosarcoma Saos2 cells.

Additional analysis of complex formation is carried out by other state-of-the-art techniques, such as chromatography, electron microscopy, immunoprecipitation in combination with Western Blot, and/or native PAGE.

In one embodiment of the method, for the above listed cells lines, which exclusively grow as adhesion cell culture, the cells are suspended in a buffer known in the art, such as PBS buffer, prior to analysis.

The physical samples for the offline assay measurements are acquired concurrently with the Raman spectra. Thereby, a direct correlation of the Raman measurement and the reference data acquired by an offline assay method is established.

The assay measurements are conventional methods known to the skilled person. These assays include, e.g., assays performed in vitro, such as immunoprecipitation, followed by Western-blot analysis, HPLC and Mass spectrometry, and qualitative and/or quantitative PCR analyses, wherein the methods may be applied alone or they are applied in combination, and cell-based assays for actual cells, such as fluorescent two-hybrid (F2H) analysis, automated cell counting comprising the determination of total cell count (TTC), and viable cell count (VCC), and the ratio of the aforementioned, wherein the measurement is based on Trypan-blue staining, or by fluorescence activated cell sorting (FACS).

## Claims

1. A composition comprising Protoporphyrin IX (PpIX) or a pharmaceutically acceptable salt thereof and a pharmaceutical excipient, wherein preferably the excipient is a carrier or a diluent.

2. The composition of claim 1 for use in treating cancer, wherein the composition is to be administered to a cancer patient to inhibit the growth of cancer cells, in particular cancer stem cells.

3. The composition for the use of claim 2, wherein the cancer is a brain tumor, preferably a glioblastoma, a pancreatic tumor, a kidney tumor, colon carcinoma, osteosarcoma, or wherein the cancer is leukemia.

4. The composition of claim 1 or the composition for the use of any one of claims 2-3, wherein the concentration of PpIX is selected from 1-10 µg/ml, preferably 10 µg/ml.

5. A composition comprising a TauT inhibitor or a pharmaceutically acceptable salt thereof for use in treating cancer, wherein the composition is to be administered to a cancer patient to inhibit the growth of cancer cells, in particular cancer tumor cells having a mutation in the TP53 gene or cancer tumor cells TP53 gene or cancer tumor cells lacking a mutation in the TP53 gene, wherein the cancer tumor cells lacking a mutation in the TP53 gene are more vulnerable to ROS-induced damage or/and damage induced by Reactive Nitrogen Species (RNS).

6. The composition for the use of claim 5, wherein the TauT inhibitor is selected from piperidine-4-sulfonic acid, g-Aminobutyric acid, g-vinyl-aminobutyric acid, β-alanine, 2-Amino-2-methyl-1-propanol (AMP) or any pharmaceutically acceptable salt thereof, preferably piperidine-4-sulfonic acid or a pharmaceutically acceptable salt thereof.

7. The composition for the use of claims 5 or 6, wherein the concentration of the TauT inhibitor, preferably piperidine-4-sulfonic acid, is selected between 0.15 mM to 15 mM.

8. The composition for the use of any one of claims 2-4, and 7 or the use of any one of claims 5-7, wherein the cancer cells are tumor cells having a mutation in the TP53 gene, wherein the mutation results in an altered expression of a p53 translation product and/or in expression of a non-functional p53 protein that corresponds to an alteration in a human p53 gene associated with tumor growth.

9. A kit for analyzing anti-tumor effect of a pharmaceutical composition inhibitor comprising
(i) a Raman spectrometer comprising a Raman probe
(ii) optionally a cell imaging apparatus,
(iii) a composition comprising an anti-tumor agent,
(iv) a single or a multi-well plate, wherein the multi-well-plate preferably is selected from a 24-, 48-, or a 96-well plate.

10. A kit according to claim 9, wherein the Raman probe is configured to automatically screen a single- or a multi-well-plate, wherein the Raman probe is connected to a transport mechanism that is electrically connected to a controller configured to move the probe relative to the single- or multi-well-plate in a horizontal plane along an x-, y-axis and vertically along the z-axis.

11. A kit of claim 9 or 10, wherein the anti-tumor agent is PpIX, preferably having a concentration of 1-10 µg/ml, preferably 10 µg/ml, or a TauT inhibitor, wherein the TauT inhibitor is selected from piperidine-4-sulfonic acid, g-Aminobutyric acid, g-vinyl-aminobutyric acid, β-alanine, or any pharmaceutically acceptable salt thereof, preferably piperidine-4-sulfonic acid or a pharmaceutically acceptable salt thereof, preferably having a concentration between 0.15 mM and 15 mM.

12. An *in vitro* method for monitoring the efficacy of anti-tumor agent, preferably PpIX or a TauT-inhibitor in real-time, wherein the method comprises:
providing a sample comprising tumor cells, preferably glioblastoma tumor cells, from a patient and cultivated as a cell culture, preferably having a mutation in the TP53 gene, wherein the mutation results in an altered expression of a *p53* translation product and/or in expression of a non-functional p53 protein that corresponds to an alteration in a human *p53* gene associated with tumor growth;
^ contacting the tumor cells with a taurine transporter (TauT) inhibitor, wherein the taurine transporter inhibitor is selected from piperidine-4-sulfonic acid, g-Aminobutyric acid, g-vinyl-aminubutyric acid, β-alanine or any pharmaceutically acceptable salt thereof, preferably piperidine-4-sulfonic acid or a pharmaceutically acceptable salt thereof,
contacting the tumor cells with protoporphyrin IX (PpIX) or a pharmaceutically acceptable salt thereof, and/or
contacting the tumor cells with an anti-cancer drug selected from a chemotherapeutic agent, and
and acquiring continuous Raman spectra of the cancer cell culture samples.

13. The method of claim 12, wherein the tumor cells, preferably glioblastoma cells, are contacted with PpIX, wherein the excitation wavelength at which the PpIX is excited is at 785 nm or at 993 nm, preferably at 993 nm.

14. The method of claim 12 or 13, wherein the Raman spectrum is carried out by a Raman flow assembly, comprising: The Raman Rxn-10 probe that transmits the Raman signal to the analyzer along an optical fiber. The micro flow cell that contains the cell culture and gives a significant amplification of the collected Raman signal, wherein the flow cell has a total volume of around 2-20 ml, preferably 4-18, more preferably 12-14 ml.
